# EUROPÄISCHE PATENTANMELDUNG

(11) **EP 3 416 073 A1**
(43) Veröffentlichungstag der Anmeldung: **19.12.2018**
(21) Anmeldenummer: 17176139.8
(22) Anmeldetag: 14.06.2017
(51) Int. Cl.: G06F 19/00, A61M 1/14

(54) **VERFAHREN ZUR ÜBERWACHUNG UND FERNBEDIENUNG EINER BLUTBEHANDLUNGSEINRICHTUNG**

(71) Anmelder: Fresenius Medical Care Deutschland GmbH, 61352 Bad Homburg (DE)
(72) Erfinder: Grießmann, Erik, 97422 Schweinfurt (DE)
(74) Vertreter: Bobbert & Partner Patentanwälte PartmbB

(57) **Zusammenfassung**

Die Erfindung betrifft ein Verfahren zur Überwachung und/oder Fernbedienung einer Blutbehandlungseinrichtung (1), die folgenden Schritte umfassend: Bereitstellen eines Anzeigegeräts (2), wobei das Anzeigegerät (2) einen Bildschirm (3) und einen Interpreter (4) aufweist, Bereitstellen von GUI-Layout-Ressourcen (8) und/oder dynamischen Daten, Interpretation der GUI-Layout-Ressourcen (8) und/oder dynamischen Daten durch den Interpreter (4) und Ausgabe einer Bildschirmanordnung durch den Interpreter (4).

## Beschreibung

Die Erfindung betrifft ein Verfahren zur Überwachung und/oder Fernbedienung einer Blutbehandlungseinrichtung. Zudem betrifft die Erfindung ein Verfahren zum Speichern von Informationen über den Zustand einer Blutbehandlungseinrichtung sowie ein Verfahren zum Rekonstruieren, insbesondere zum exakten Rekonstruieren, einer oder einer Abfolge mehrerer Bildschirmanordnungen aus einer Blutbehandlungseinrichtung. Darüber hinaus umfasst die Erfindung ein Anzeigegerät und eine Blutbehandlungseinrichtung zur Ausführung des erfindungsgemäßen Verfahrens, ein digitales Speichermedium, ein Computerprogramm-Produkt und ein Computerprogramm.

Bei bekannten Verfahren zur Überwachung und/oder Fernbedienung einer Blutbehandlungseinrichtung, zum Speichern von Informationen über den Zustand einer Blutbehandlungseinrichtung und zum Rekonstruieren einer Abfolge mehrerer Bildschirmanordnungen aus einer Blutbehandlungseinrichtung müssen häufig erhebliche Datenmengen gespeichert und/oder übertragen werden. Eine Reduzierung der benötigten Datenmengen kann in einigen Fällen vorteilhaft sein.

Die Aufgabe der vorliegenden Erfindung ist es, ein weiteres Verfahren zur Überwachung und/oder Fernbedienung, zum Speichern von Informationen über den Zustand der Blutbehandlungseinrichtung und zum Rekonstruieren einer Abfolge mehrerer Bildschirmanordnungen aus einer Blutbehandlungseinrichtung bereitzustellen. Zudem soll eine weitere Blutbehandlungseinrichtung und ein Anzeigegerät zur Ausführung eines derartigen Verfahrens, ein geeignetes digitales Speichermedium, ein geeignetes Computerprogramm-Produkt und ein geeignetes Computerprogramm bereitgestellt werden.

Die Aufgabe kann durch die Verfahren nach Anspruch 1, 6 und 8 sowie durch das Anzeigegerät und die Blutbehandlungseinrichtung nach Anspruch 11 beziehungsweise 12 gelöst werden. Zudem wird die Aufgabe durch ein geeignetes digitales Speichermedium, ein geeignetes Computerprogramm-Produkt und ein geeignetes Computerprogramm nach den Ansprüchen 13 bis 15 gelöst.

Das erfindungsgemäße Verfahren zur Überwachung und/oder Fernbedienung, insbesondere einer Blutbehandlungseinrichtung, umfasst die folgenden Schritte:
- Bereitstellen eines Anzeigegeräts, wobei das Anzeigegerät einen Bildschirm und einen Interpreter aufweist
- Bereitstellen von GUI-Layout-Ressourcen und/oder dynamischen Daten
- Interpretation der GUI-Layout-Ressourcen und/oder dynamischen Daten durch den Interpreter
- Ausgabe einer Bildschirmanordnung durch den Interpreter.

Wenn hierin von Blutbehandlungseinrichtungen die Rede ist, so sind damit Einrichtungen gemeint, die zur Behandlung von Blut geeignet, insbesondere dazu konfiguriert sind. Darüber hinaus sind im Sinne der Anmeldungen jedoch auch alle Medizingeräte von dem Begriff Blutbehandlungseinrichtungen umfasst, insbesondere derartige Medizingeräte, die zur Ausführung des erfindungsgemäßen Verfahrens geeignet und/oder konfiguriert sind, sofern dies nicht zu für den Fachmann erkennbaren Widersprüchen führt.

Eine Bildschirmanordnung ist eine Anordnung von Objekten, die auf einem Bildschirm angezeigt werden können und/oder sollen. Derartige Objekte können beispielsweise Texte, Grafiken und Fotos umfassen.

Eine Bildschirmanordnung kann durch einen geeigneten Bildschirm angezeigt werden. Die Bildschirmanordnung kann in einem Format vorliegen, die unmittelbar durch Bildschirm angezeigt werden kann oder muss in einigen Fällen erst in ein solches Format oder ein geeignetes Signal übersetzt werden.

Bildschirmanordnungsdaten sind Daten, die es erlauben, Bildschirmanordnungen zu erstellen und/oder zu rekonstruieren, gegebenenfalls durch Zugriff auf von der konkreten Behandlung unabhängige Informationen.

Überwachen bedeutet hierin das passive Anzeigen von Informationen, während Fernbedienung eine Interaktion ermöglicht. Bei einer Fernbedienung kann beispielsweise eine Eingabe am Anzeigegerät erfolgen, woraufhin Informationen über die Eingabe an die Blutbehandlungseinrichtung übertragen werden können.

GUI steht hier für graphische Benutzerschnittstelle (graphical user interface).

GUI-Layout-Ressourcen sind Informationen, mit deren Hilfe der Interpreter eine oder mehrere Bildschirmanordnungen erstellen kann und die in der Regel unabhängig von den Parametern, Benutzerinteraktionen und Messwerten einer konkreten Behandlung sind. Die GUI-Layout-Ressourcen können beispielsweise als Text oder binär vorliegen. In einigen Ausführungsformen können die GUI-Layout-Ressourcen auch in einer Datenbank verwaltet werden.

Dynamische Daten sind Daten, die vorzugsweise zu den GUI-Layout-Ressourcen komplementär sind und/oder in den GUI-Layout-Ressourcen nicht vorhanden sind. Dynamische Daten können beispielsweise Daten sein, die erst im Betrieb der Blutbehandlungseinrichtung erzeugt werden und/oder sich in der Regel von Patient zu Patient und/oder Sitzung zu Sitzung unterscheiden, wie z.B. Patientenname, Gewicht, Geburtsdatum, aktuelles Datum und aktuelle Uhrzeit. Dies kann beispielsweise bei Messdaten und Verbrauchswerten der Fall sein. Dynamische Daten können auch Daten sein, die in der Regel nicht veränderlich sind, wie z.B. Texte einer Gebrauchsanweisung.

In einigen Ausführungsformen sind dynamische Daten z.B. eine Nummer/eindeutige Identifikation eines Bildschirms oder eine Auswahl aus einer Liste. Die Zuordnungen, welches Listenelement ausgewählt wurde, können in einigen Ausführungsformen dynamische Daten darstellen. In einigen Ausführungsformen stellen Alarme und/oder Alarmmeldungen dynamische Daten dar. Vorzugsweise sind dabei alle möglichen Alarmmeldungen bekannt. Beim Auftreten eines Alarms kann z.B. ein Alarmtext dem Alarm zugeordnet werden, beispielsweise über eine Alarmnummer. In einigen Ausführungsformen stellen ein oder mehrere der folgenden Werte dynamische Daten dar: das momentane Einlauf- und/oder Auslaufvolumen, die momentane Verweilzeit und Daten aus der Gerätekonfiguration wie beispielsweise Lautstärke, angezeigte Sprache, Bildschirmhelligkeit etc.

Dynamische Daten können beispielsweise abgespeichert sein (z.B. in einer Datei) und/oder über eine Verbindung (z.B. eine kabelgebundene oder eine kabellose Verbindung, z.B. Netzwerkverbindung) übertragen werden, insbesondere an das Anzeigegerät, insbesondere von der Blutbehandlungseinrichtung.

Die Übertragung oder Bereitstellung der dynamischen Daten erfolgt dabei in einigen Ausführungsformen über eine kabelgebundene Verbindung oder eine kabellose Verbindung, insbesondere ein kabelgebundenes oder ein kabelloses Netzwerk. Dynamische Daten können auch in einer Konfigurationsdatei gespeichert sein, beispielsweise wenn feste Bildschirmvarianten erzeugt werden sollen, z.B. um Informationen einer Gebrauchsanweisung darzustellen.

In einigen Ausführungsformen umfassen die GUI-Layout-Ressourcen statische GUI-Elemente und dynamische GUI-Elemente.

Statische GUI-Elemente stellen dabei konstante Elemente dar, die sich bei der Anzeige auf einem Bildschirm nicht ändern, dies gilt vorzugsweise über die gesamte Zeit, in der der Bildschirm dargestellt wird. Statische GUI-Elemente können beispielsweise Hintergrundbilder und/oder feste Texte sein. Feste Texte können dabei in verschiedenen Sprachvarianten vorliegen.

Die GUI-Layout-Ressourcen enthalten beispielsweise Formatierungen, Texte und/oder Grafiken. Formatierungen umfassen beispielsweise Schriftarten, Schriftgrößen und/oder Textalignment. Zudem können die GUI-Layout-Ressourcen Positionsinformationen umfassen, die beispielsweise die Positionen von Texten und/oder Grafiken bestimmen.

Die GUI-Layout-Ressourcen können Varianten für unterschiedliche Sprachen umfassen. Die Varianten können sich beispielsweise in den Texten, Formatierungen und/oder Positionsinformationen unterscheiden. Unterschiedliche Sprachen erfordern gegebenenfalls unterschiedliche Formatierungen und gegebenenfalls auch eine andere Anordnung der graphischen Elemente. Dies kann beispielsweise bei arabischen Sprachen mit einer Leserichtung von rechts nach links erforderlich sein. In diesem Fall können Bilder beispielsweise in einem Menü rechts- statt linksbündig angeordnet werden. Die für einige Sprachen abweichende Formatierung und/oder Anordnung der graphischen Elemente kann beispielsweise aus einer Konfigurationsdatei ausgelesen werden. Dies bedeutet, dass in einigen Ausführungsformen auch eigentlich sprachunabhängige Ressource-Dateien für manche Sprachen zusätzlich eingelesen werden. Im genannten Beispiel bei arabischer Sprache sind dies beispielsweise Bild-Dateien, die auch Positionsinformationen enthalten.

Dynamische GUI-Elemente sind Elemente, die abhängig von einer Bedingung unterschiedliche Ausprägungen besitzen können.

Dynamische GUI-Elemente können beispielsweise bei einem Menü vorliegen, bei dem ein Benutzer einen oder mehrere Menüeinträge auswählen kann. Weitere dynamische GUI-Elemente liegen beispielsweise bei Tasten vor, wobei die Ausprägung davon abhängen kann, welchen Zustand eine Taste hat. In einigen Ausführungsformen kann eine Taste die möglichen Zustände "gedrückt" oder "nicht gedrückt" aufweisen, alternativ kann in einigen Ausführungsformen eine Taste zudem den möglichen Zustand "deaktiviert" oder "unsichtbar" aufweisen. Ferner besteht die Möglichkeit, dass sich ein Button wie ein Schalter, welcher, wenn er gedrückt wird, in diesem Zustand verweilt, oder wie eine Taste verhält, welche hingegen wieder in den nicht-gedrückten Zustand zurückkehrt.

Dynamische GUI-Elemente können beispielsweise variable Werte umfassen. Derartige variable Werte können beispielsweise Zustände, berechnete Werte und/oder Messwerte eines Geräts, insbesondere einer Blutbehandlungseinrichtung, darstellen. Derartige variable Werte können z.B. von einem dynamischen Prozess ermittelt werden, z.B. ein momentan gefördertes Volumen oder ein gemessenes Gewicht. Variable Werte können z.B. durch einen Benutzer eingegebene oder ausgewählte Werte darstellen.

Dynamische GUI-Elemente können beispielsweise Animationen (z.B. die momentane Bildnummer einer Bildersequenz) und/oder Fortschrittsbalken umfassen.

Die Anzahl der Varianten von Bildschirmanordnungen ist abhängig von der Anzahl der dynamischen GUI-Elemente und von der Anzahl von Zuständen, die jedes dynamische GUI-Element einnehmen kann. Beispielsweise kann ein scrollbares Menü mit drei Menüelementen sechs Varianten aufweisen, da jedes Menüelement auf eine vorgegebene Position gescrollt werden kann und das jeweilige Menüelement beispielsweise einen Button mit zwei Zuständen (z.B. "gedrückt" und "nicht gedrückt") aufweist. Die Anzahl der Varianten kann gegebenenfalls auch höher sein, wenn z. B. aufgrund eines Berechtigungsmanagements gewisse Menüelemente deaktiviert sind.

Die Anzahl der möglichen Varianten von Bildschirmanordnungen ist eine Maßzahl für die dynamische Komplexität einer Bildschirmanordnung und/oder der Darstellung der Bildschirmanordnung. Wenn beispielsweise variable Zahlenwerte (z.B. ein Datum) mit gleicher Formatierung angezeigt werden, so erhöht dies nicht die Komplexität einer Bildschirmanordnung und/oder der Darstellung der Bildschirmanordnung. Falls jedoch z.B. ein Kalender mit Monatsnamen dargestellt wird, so existieren zwölf Varianten von Bildschirmanordnungen und/oder von Darstellungen der Bildschirmanordnung. Die dynamische Komplexität ist in einigen Ausführungsformen von Bedeutung, um das Layout von Bildschirmen korrekt gestalten zu können. Verschiedene Zahlenwerte eines Datums dürfen dabei beispielsweise nicht dazu führen, dass es zu Überschneidungen von GUI-Elementen kommt.

Übersetzungstexte können in einigen Ausführungsformen Teil der GUI-Layout-Ressourcen sein. In einigen Ausführungsformen sind Übersetzungstexte zusätzlich oder alternativ Teil der dynamischen Daten oder in einer weiteren Einheit, z.B. einer Datenbank für Übersetzungstexte, gespeichert.

Die Informationen, die die GUI-Layout-Ressourcen und die dynamischen Daten gemeinsam bereitstellen, reichen vorzugsweise aus, um eine Bildschirmanordnung zu erstellen und auszugeben.

Um eine Bildschirmanordnung einer Blutbehandlungseinrichtung auf einem entfernten Anzeigegerät anzuzeigen, müssen lediglich die dynamischen Daten übertragen werden, sofern das Anzeigegerät auf die GUI-Layout-Ressourcen zugreifen kann. Bei gegebenen GUI-Layout-Ressourcen ist vorzugsweise jedem Satz an dynamischen Daten eine Bildschirmanordnung eindeutig zugeordnet.

Der Interpreter kann erfindungsgemäß aus GUI-Layout-Ressourcen und dynamischen Daten eine oder mehrere Bildschirmanordnungen erzeugen. Der Interpreter ist z.B. eine Software, die in einer Recheneinheit des Anzeigegeräts ausgeführt wird.

In einigen Ausführungsformen ist das erfindungsgemäße Verfahren zur Überwachung derart ausgestaltet, dass durch den Interpreter Bildschirmanordnungen erzeugt werden, die identisch sind mit den Bildschirmanordnungen, die auf der Blutbehandlungseinrichtung zeitgleich oder annähernd zeitgleich angezeigt werden.

In einigen Ausführungsformen wird zur Anzeige von Bildschirmanordnungen auf der Blutbehandlungseinrichtung derselbe Mechanismus und/oder Interpreter wie auf dem Anzeigegerät verwendet.

In einigen Ausführungsformen werden auf der Blutbehandlungseinrichtung und/oder dem Anzeigegerät binäre GUI-Layout-Ressourcen verwendet. Die GUI-Layout-Ressourcen lassen sich vorzugsweise in XML-Daten umwandeln. In einigen Ausführungsformen ist eine Umwandlung auch in die andere Richtung möglich.

In einigen Ausführungsformen liegen die GUI-Layout-Ressourcen in definierter Form auf der Blutbehandlungseinrichtung vor. In einigen Ausführungsformen können die GUI-Layout-Ressourcen durch das Anzeigegerät heruntergeladen werden. Alternativ können die GUI-Layout-Ressourcen von einem Server verwaltet werden. Dabei kann z. B. die Blutbehandlungseinrichtung und/oder das Anzeigegerät in Bezug auf die GUI-Layout-Ressourcen mit dem Server synchronisiert werden. Aktualisierungen, insbesondere der GUI-Layout-Ressourcen, können in einigen Ausführungsformen beispielsweise über den Server auf die Blutbehandlungseinrichtung und/oder das Anzeigegerät übertragen werden.

Das erfindungsgemäße Verfahren zum Speichern von Informationen umfasst die folgenden Schritte:
- Bereitstellen einer Blutbehandlungseinrichtung mit einer Steuereinheit und einem Bildschirm
- Bereitstellen eines maschinenlesbaren Trägers und/oder eines Netzwerkes
- Speichern von Informationen über den Zustand der Blutbehandlungseinrichtung auf dem maschinenlesbaren Träger und/oder Übertragen der Daten in das Netzwerk.

Dem vorliegenden Verfahren entsprechend speichert eine Blutbehandlungseinrichtung Informationen über ihren Zustand auf einem maschinenlesbaren Träger oder überträgt diese Informationen über ein Netzwerk.

Zudem umfasst die Erfindung das Verfahren zum Rekonstruieren, insbesondere zum exakten Rekonstruieren, einer oder einer Abfolge mehrerer Bildschirmanordnungen aus einer Blutbehandlungseinrichtung mit den folgenden Schritten:
- Bereitstellen eines Visualisierers
- Bereitstellen von Bildschirmanordnungsdaten von einem maschinenlesbaren Träger und/oder aus einem Netzwerk
- Verarbeiten der Bildschirmanordnungsdaten durch den Visualisierer
- Ausgabe einer Bildschirmanordnung.

In einigen Ausführungsformen kann der Visualisierer Bildschirmanordnungsdaten derart verarbeiten, dass eine Bildschirmanordnung entsteht. Ein Visualisierer ist in einigen Ausführungsformen eine Software oder eine dedizierte Hardware, die aus Bildschirmanordnungsdaten eine Bildschirmanordnung erstellen, insbesondere rekonstruieren, kann.

Zur Überprüfung des exakten Rekonstruierens einer Bildschirmanordnung kann in einigen Ausführungsformen durch das Anzeigegerät eine Prüfsumme oder ein Hashwert erzeugt werden und dieser auf dem Anzeigegerät und/oder auf der Blutbehandlungseinrichtung überprüft werden. Vorzugsweise werden sowohl auf der Blutbehandlungseinrichtung als auch auf dem Anzeigegerät eine Prüfsumme bzw. ein Hashwert erzeugt und diese miteinander auf der Blutbehandlungseinrichtung und/oder dem Anzeigegerät verglichen. Falls die Prüfsummen bzw. Hashwerte identisch sind, so wird dies vorzugsweise als Beleg gewertet, dass die Bildschirmanordnung exakt, insbesondere Pixel für Pixel, rekonstruiert wurde.

Die Bildschirmanordnungsdaten enthalten vorzugsweise nur das Minimum an notwendigen Informationen, um eine Bildschirmanordnung zu rekonstruieren. Um die erforderliche Datenmenge zur Rekonstruktion einer Bildschirmanordnung zu verringern, kann der Visualisierer in einigen Ausführungsformen auf Ressourcen zugreifen, die behandlungsunabhängige Informationen zum Erzeugen von Bildschirmanordnungen aufweisen. Die GUI-Layout-Ressourcen sind vorzugsweise am Anzeigegerät vorhanden. Für die Rekonstruktion der Bildschirmanordnung werden dann in einigen Ausführungsformen nur noch die Bildschirmnummer sowie die dynamischen Daten benötigt.

In einigen Ausführungsformen ist der Visualisierer ein Teil der Blutbehandlungseinrichtung und/oder des Anzeigegeräts.

Die Erfindung umfasst zudem eine Blutbehandlungseinrichtung und ein Anzeigegerät zum Ausführen des erfindungsgemäßen Verfahrens. Die Blutbehandlungseinrichtung und/oder das Anzeigegerät weisen vorzugsweise mindestens einen Bildschirm auf, vorzugsweise einen Touchscreen oder einen Bildschirm mit einer zugehörigen Tastenanordnung zur Benutzerinteraktion.

Ein erfindungsgemäßes digitales, insbesondere nichtflüchtiges, Speichermedium, insbesondere in Form eines maschinenlesbaren Trägers, insbesondere in Form einer Diskette, CD, DVD oder EPROM, insbesondere mit elektronisch oder optisch auslesbaren Steuersignalen, kann derart mit einem programmierbaren Computersystem zusammenwirken, dass die maschinellen Schritte eines erfindungsgemäßen Verfahrens veranlasst werden.

Ein erfindungsgemäßes Computerprogramm-Produkt weist einen volatilen, flüchtigen oder auf einem maschinenlesbaren Träger gespeicherten Programmcode oder eine Signalwelle zur Veranlassung der maschinellen Schritte des erfindungsgemäßen Verfahrens, wenn das Computerprogramm-Produkt auf einem Rechner abläuft, auf. Unter einem Computerprogramm-Produkt kann erfindungsgemäß beispielsweise ein auf einem Träger gespeichertes Computerprogramm, ein Embedded System als umfassendes System mit einem Computerprogramm (z. B. elektronisches Gerät mit einem Computerprogramm), ein Netzwerk von computerimplementierten Computerprogrammen (z. B. Client/Serversystem, Cloud Computing System, etc.), oder ein Computer, auf dem ein Computerprogramm geladen ist, abläuft, gespeichert ist, ausgeführt oder entwickelt wird, verstanden werden.

Der Begriff "maschinenlesbarer Träger", wie er hierin verwendet wird, bezeichnet in manchen beispielhaften Ausführungsformen der vorliegenden Erfindung einen Träger, der von Software und/oder Hardware interpretierbare Daten oder Informationen enthält. Der Träger kann ein Datenträger, wie eine Diskette, eine CD, DVD, ein USB-Stick, eine Flashcard, eine SD-Karte und dergleichen sein.

Ein erfindungsgemäßes Computerprogramm weist einen Programmcode auf zur Veranlassung der maschinellen Schritte des erfindungsgemäßen Verfahrens, wenn das Computerprogramm auf einem Computer abläuft. Unter einem Computerprogramm kann erfindungsgemäß beispielsweise ein physikalisches, vertriebsfähiges Software-Produkt verstanden werden, welches ein Programm aufweist.

Für das erfindungsgemäße digitale Speichermedium, das erfindungsgemäße Computerprogramm-Produkt und das erfindungsgemäße Computerprogramm gilt, dass alle, einige oder manche der maschinell durchgeführten Schritte des erfindungsgemäßen Verfahrens veranlasst werden. Dies gilt insbesondere im Zusammenwirken mit einem erfindungsgemäßen Anzeigegerät und einer erfindungsgemäßen Blutbehandlungseinrichtung.

Wenn hierin von Anzeigegeräten gesprochen wird, so sind damit vorzugsweise Anzeigegeräte gemeint, die unabhängig von der Blutbehandlungseinrichtung angeordnet sind oder angeordnet werden können.

Eine Anzeige, die fest mit der Blutbehandlungseinrichtung verbunden ist, wird hierin vorzugsweise als Bildschirm der Blutbehandlungseinrichtung bezeichnet.

Blutbehandlungseinrichtung, Blutbehandlungsmaschine und Blutbehandlungsgerät werden hierin als Synonyme verwendet. Auch Anzeigegerät, Anzeigeeinrichtung und Anzeigeeinheit sind als Synonyme zu verstehen.

Die erfindungsgemäße Blutbehandlungseinrichtung oder Blutbehandlungsmaschine kann zur Dialyse, Hämodialyse, Hämodiafiltration, Filtration oder Aphärese verwendbar sein.

Bei allen Ausführungen hierin ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Wann immer hierin Zahlenworte genannt werden, so versteht der Fachmann diese als Angabe einer zahlenmäßig unteren Grenze.

Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wenn hierin von einer Einrichtung die Rede ist, die zum Ausführen eines Verfahrensschritts dient, so kann die betreffende Einrichtung zum Ausführen dieses Schritts jeweils konfiguriert, programmiert, vorgesehen und/oder ausgestaltet sein.

Erfindungsgemäße Ausführungsformen können eines oder mehrere der vorstehend oder im Folgenden genannten Merkmale aufweisen. Dabei können die hierin genannten Merkmale in beliebigen Kombinationen Gegenstand von erfindungsgemäßen Ausführungsformen sein, sofern der Fachmann eine konkrete Kombination nicht als technisch unmöglich erkennt. Erfindungsgemäße Ausführungsformen sind ferner Gegenstand der Unteransprüche.

In einigen Ausführungsformen werden durch den Interpreter erstellte Bildschirmanordnungen auf einem Bildschirm angezeigt, insbesondere auf einem Bildschirm des Anzeigegeräts und/oder der Blutbehandlungseinrichtung.

Die GUI-Layout-Ressourcen können in einigen Ausführungsformen auf dem Anzeigegerät vorgehalten und/oder über ein mit dem Anzeigegerät verbundenes Gerät oder Netzwerk bereitgestellt werden.

In einigen Ausführungsformen kann bei dem Verfahren zum Rekonstruieren einer oder einer Abfolge mehrerer Bildschirmanordnungen aus einer Blutbehandlungseinrichtung die rekonstruierte Bildschirmanordnung in einer anderen Sprachversion angezeigt werden als der aufzeichnenden Blutbehandlungseinrichtung.

So kann beispielsweise ein Anwendungsberater das Gerät in seiner eigenen Sprache betreiben und eine Blutbehandlungseinrichtung, die in einer für den Anwendungsberater fremden Sprache betrieben wird, bedienen.

In einigen Ausführungsformen können mehrere Anzeigegeräte zeitgleich oder annähernd zeitgleich dieselbe Bildschirmanordnung der Blutbehandlungseinrichtung anzeigen. In einigen Ausführungsformen können die Bildschirmanordnungen von mehreren Blutbehandlungseinrichtungen gleichzeitig auf einem Bildschirm eines Anzeigegeräts angezeigt werden.

In einigen Fällen kann es erforderlich sein, die Texte einer Bildschirmanordnung in eine oder mehrere Sprachen zu übersetzen, dabei können beispielsweise mehrere oder alle Bildschirm-Varianten der übersetzten Sprache gespeichert werden, insbesondere in einem Bilddateiformat (z.B. PNG, JPG, GIF, TIFF usw.). Mit einer geeigneten (Übersetzungs-)Software können Formatierungen (z.B. Position und Schriftgröße) und Textüberschneidungen überprüft, verifiziert und gegebenenfalls verändert werden, ohne dass eine Sprachvariante auf eine Blutbehandlungseinrichtung aufgespielt werden muss.

Eine Sprachvariante kann beispielsweise auch online übersetzt werden, d.h. bei Änderung eines Textes wird sofort die Darstellung eines Bildschirms aktualisiert. Formatierungsfehler und Textüberschneidungen können auf diese Weise unmittelbar sichtbar werden und entsprechend korrigiert werden.

In einer Ausführungsform wird ein für Erstellen und/oder Überprüfen einer Übersetzung geeignetes Programm (Übersetzungsmodul) mit Hilfe von Plug-In-Technologie implementiert. Dabei können die GUI-Layout-Ressourcen und der Kern des Interpreters auf dem jeweiligen Zielgerät (insbesondere der Blutbehandlungseinrichtung) und dem genannten Programm identisch sein. In einigen Ausführungsformen ist es möglich, dass die durch eine Übersetzungssoftware dargestellten Bildschirmanordnungen identisch sind mit den auf dem Zielgerät dargestellten Bildschirmanordnungen, so dass Prüfungen der Bildschirmanordnungen am Zielgerät reduziert werden oder ganz entfallen können.

In einigen Ausführungsformen können Übersetzungen auch beispielsweise über ein Web-Interface, als HTML5-Smartphone-App (Browser App) oder mit einer ähnlichen Technologie angefertigt und/oder überprüft werden. In diesen Fällen könnte ein Übersetzer beispielsweise die übersetzten Texte in einen Browser eingeben. Ein Webserver kann dann z.B. die resultierende Bildschirmanordnung generieren, so dass der Übersetzer ein unmittelbares Feedback erhalten kann. Vorteilhafterweise ist somit in einigen Fällen kein zusätzliches Tool für Übersetzungen mehr nötig und Updates stehen unmittelbar für alle Übersetzer zur Verfügung. Zudem ist es in einigen Fällen möglich, dass Übersetzungen von Bildschirmanordnungen nicht mehr über Dateien ausgetauscht werden müssen, sondern direkt auf dem Webserver gespeichert werden können und von dort heruntergeladen werden können. Auf diese Weise ist es in einigen Fällen möglich, auf eine Installation von Tools, die gegebenenfalls Administratorrechte voraussetzt, und auf eine Wartung der Tools (inklusive dem Verteilen von Updates) zu verzichten.

Die durch geeignete Software erstellten Bilddateien von Bildschirmanordnungen in ihrer jeweiligen Sprachversion können in einigen Fällen in Gebrauchsanweisungen verwendet werden.

In einigen Ausführungsformen erlaubt die Erfindung eine Überwachung. Dabei ist zumindest eine unidirektionale Verbindung zwischen Datenquelle, z.B. der Blutbehandlungseinrichtung, und dem Anzeigegerät, z.B. einem Überwachungsbildschirm, erforderlich. Eine derartige unidirektionale Verbindung kann beispielsweise kabelgebunden (beispielsweise über eine direkte oder indirekte Kabelverbindung) oder kabellos (beispielsweise über eine Funkverbindung oder Infrarotverbindung) ausgeführt sein.

Alternativ kann eine bidirektionale Verbindung eingesetzt werden. Eine bidirektionale Verbindung kann ebenfalls kabelgebunden (beispielsweise über eine direkte oder indirekte Kabelverbindung oder über ein kabelgebundenes Netzwerk, beispielsweise Ethernet, insbesondere über das Internet) oder kabellos ausgeführt sein.

Eine kabellose bidirektionale Verbindung zwischen einer Datenquelle und einer Anzeigegerät kann beispielsweise direkt zwischen Datenquelle und Anzeigegerät über elektromagnetische Wellen, insbesondere Radiofrequenz, insbesondere Bluetooth, oder über Infrarotlicht ausgeführt sein. In einigen Ausführungsformen kann eine bidirektionale Verbindung zwischen Datenquelle und Anzeigegerät über ein drahtloses Netzwerk geführt sein, beispielsweise über Wifi, Mobilfunk (z.B. GPRS, EDGE, 3G oder LTE) oder ein Mesh-Network.

Bei einer drahtlosen Verbindung können in einigen Ausführungsformen Teile des Datenverkehrs über ein kabelgebundenes Netzwerk stattfinden, beispielsweise werden die Datenquelle und/oder das Anzeigegerät über Wifi oder Mobilfunk ans Internet angebunden.

In einigen Ausführungsformen kann beispielsweise ein Arzt eine medizinische Behandlung, insbesondere eine Blutbehandlung, über ein ortsfestes oder tragbares Anzeigegerät, beispielsweise ein Smartphone, Laptop oder einen stationären Computer, überwachen. Der Bildschirm der Blutbehandlungseinrichtung und des Anzeigegeräts zur Fernüberwachung sind vorzugsweise synchron oder annähernd synchron.

In einigen Ausführungsformen der Überwachung wird ein Benutzer ausschließlich oder zusätzlich beim Auftreten bestimmter Ereignisse durch das Anzeigegerät informiert (z. B. beim Auftreten einer Warnung oder eines Alarms an der überwachten Blutbehandlungseinrichtung). Dabei kann beispielsweise der Behandlungsablauf nachträglich auf dem Anzeigegerät rekonstruiert werden. Beispielsweise kann der graphische Behandlungsverlauf (z. B. die durchlaufenen Bildschirmanordnungen seit Behandlungsbeginn) und/oder weitere Zusatzinformationen, wie z. B. ein Behandlungsprotokoll, das z. B. während des Bildablaufs beispielsweise graphisch angezeigt wird, beim Auftreten von Alarmen auf dem Anzeigegeräts zur Überwachung angezeigt werden. Ebenso können in einigen Fällen technische Information angezeigt werden, beispielsweise darüber, wie viel Lösung sich in den Beuteln befindet, Informationen über den Blutfluss oder andere Sensordaten, die beispielsweise wegen der Displaygröße der Blutbehandlungseinrichtung nicht dort angezeigt werden können oder sollen, wenn sich beispielsweise ein Patient an der Blutbehandlungseinrichtung befindet.

Falls ein Techniker die Ursache eines aufgetretenen Alarms untersuchen möchte, könnte dazu in einigen Ausführungsformen zusätzlich eine Wissensdatenbank abgefragt werden, die weitere Auskünfte zu einem bestimmten Alarm erteilt.

In einigen Ausführungsformen kann eine derartige Fernüberwachung vorteilhafterweise durch Anwendungsberater verwendet werden, die so Zugang zu Geräte- und Behandlungsinformationen haben und einem Benutzer z.B. über das Telefon genaue Anweisung zur Bedienung des Geräts geben, die dann wieder durch den Anwendungsberater kontrolliert werden können.

Zudem kann es in einigen Ausführungsformen von Vorteil sein, Blutbehandlungseinrichtungen während eines Gerätetests aus der Ferne überwachen zu können. Insbesondere wäre dies bei Tests unter harschen Bedingungen, wie z.B. in einer Klimakammer, von Vorteil.

In einigen Ausführungsformen kann an einem Anzeigegerät zur Überwachung eine Bildschirmanordnung in einer anderen Sprachversion abgerufen werden als die Sprachversion der ursprünglichen Bildschirmanordnung auf der Blutbehandlungseinrichtung.

Vorzugsweise sind Sprachinformationen Teil der GUI-Layout-Ressourcen. Jeder Text besitzt vorzugsweise einen eindeutigen Identifikationscode, welcher auf eine Alarmmeldung verweist. Für die Darstellung des Alarms in einer Bildschirmanordnung wird vorzugsweise der Alarmtext, der dem Identifikationscode entspricht, aus in den GUI-Layout-Ressourcen vorhandenen Sprachtexten geladen. Da auch Positionen und Formatierungen sprachabhängig sein können, werden auch diese vorzugsweise zur Darstellung aus den GUI-Layout-Ressourcen geladen, wofür es eine GUI-Ressourcen-Konfiguration für jede Sprache geben kann.

Die übertragenen dynamischen Daten sind vorzugsweise sprachunabhängig. Jede Sprache verwendet dabei vorzugsweise die GUI-Layout-Ressourcen einer Mastersprache (z. B. Bildinformation, Screenaufbaubeschreibung).

In einigen Ausführungsformen müssen nur sprachabhängige Elemente wie Texte, Positionen und/oder Formatierungen für eine geänderte oder unterschiedliche Sprache ausgetauscht werden. Die GUI-Layout-Ressourcen müssen dafür vorzugsweise entweder einmalig von der Blutbehandlungseinrichtung oder einem anderen Ort heruntergeladen werden, beispielsweise von einem Webserver, von einer Datenbank etc.

Das Anzeigegerät zur Überwachung hat dazu beispielsweise Übersetzungstexte gespeichert, auf die es zurückgreifen kann.

In einigen Ausführungsformen hat das Anzeigegerät Zugriff auf Übersetzungstexte außerhalb des Anzeigegeräts, beispielsweise über ein Netzwerk. Alternativ werden in den von der Blutbehandlungseinrichtung an das Anzeigegerät zur Überwachung übertragenen Daten eine oder mehrere Sprachversionen übermittelt, die sich von der auf der Blutbehandlungseinrichtung eingestellten Sprachversion unterscheiden.

In bestimmten Ausführungsformen weisen die übertragenen Daten keine Sprachversion auf, jeder Text weist lediglich einen Code (der über alle Sprachversionen identisch ist) oder Hashwert (z.B. bezogen auf eine Sprache) auf, der auf dem jeweiligen Anzeigegerät in eine vorzugsweise einstellbare Sprachversion übersetzt wird.

Alternativ enthalten die übertragenen Daten eine Sprachversion und ein Anzeigegerät kann auf Übersetzungstexte, beispielsweise in einer Datenbank, die sich auf dem Anzeigegerät befindet oder über ein Netzwerk zugänglich ist, zugreifen.

Vorzugsweise ist die Sprachversion auf dem Anzeigegerät einstellbar. Ein Anwendungsberater könnte einem Patienten assistieren, selbst wenn der Patient seine Blutbehandlungseinrichtung auf eine für den Berater fremde Sprache eingestellt hat, indem der Berater die Sprache an seinem Anzeigegerät ändert.

In einigen Fällen könnte somit das Anzeigegerät zur Fernüberwachung als ein automatischer Übersetzer der graphischen Benutzerschnittstelle, insbesondere der Bildschirmanordnungen, dienen.

In einigen Fällen kann als Anzeigegerät zur Überwachung ein Browser, der auf einem Computer (z.B. einem stationären Computer oder einem tragbaren Computer, wie z.B. einem Tablet-Computer oder einem Smartphone) ausgeführt wird, dienen. Zu diesem Zweck kann in einigen Ausführungsformen die Blutbehandlungseinrichtung mit einem Webserver kommunizieren oder einen Webserver enthalten.

In einigen Ausführungsformen ist das Anzeigegerät für die Überwachung dazu geeignet und/oder konfiguriert, mehrere Blutbehandlungseinrichtungen zu überwachen. In einigen Ausführungsformen können mehrere Blutbehandlungseinrichtungen in einem Browserfenster oder in einer App überwacht werden.

In einigen Ausführungsformen ist es so möglich, dass beispielsweise eine Person alle Blutbehandlungseinrichtungen einer Station auf einen Blick überwachen kann. Alternativ oder zusätzlich können in einigen Ausführungsformen die Systemtests in der Produktion von Blutbehandlungseinrichtungen bei mehreren Blutbehandlungseinrichtungen zugleich überwacht werden.

In einigen Ausführungsformen sind die Blutbehandlungseinrichtung und das Anzeigegerät geeignet und/oder konfiguriert dazu, dass die Blutbehandlungseinrichtung von dem Anzeigegerät fernbedient werden kann. Zur Fernbedienung wird in der Regel eine bidirektionale Verbindung zwischen der Blutbehandlungseinrichtung und dem Anzeigegerät benötigt, vorzugsweise eine bidirektionale Verbindung wie hierin bereits im Zusammenhang mit der entfernten Überwachung beschrieben ist.

Ein Anzeigegerät weist in einigen Ausführungsformen ein oder mehrere Eingabeelemente auf. Eingabeelemente können insbesondere physische Tasten und/oder Schalter sein, insbesondere Tasten, die um den Bildschirm herum angeordnet sind. Als Eingabeelement kann auch ein Touchscreen bezeichnet werden.

In einigen Ausführungsformen umfasst das Verfahren das Übertragen einer von einem Bediener über das Eingabeelement des Anzeigegeräts getätigten Eingabe an die Blutbehandlungseinrichtung. Zur Fernbedienung kann beispielsweise das Betätigen eines Bedienelements auf dem Anzeigegerät an die Blutbehandlungseinrichtung übertragen werden.

Wenn das Bedienelement eine Taste oder ein Schalter ist, dann kann eine Identifikation der Taste oder des Schalters und der Zustand/die Zustandsänderung der Taste oder des Schalters an die Blutbehandlungseinrichtung übertragen werden. Die Blutbehandlungseinrichtung kann dann die Eingabe des Bedieners interpretieren und z.B. programmierte Vorgänge einleiten.

In einigen Ausführungsformen des Anzeigegeräts mit Touchscreen können beispielsweise bei einem Berühren des Touchscreens die Koordinaten der Berührung an die Blutbehandlungseinrichtung übertragen werden. Die Blutbehandlungseinrichtung kann dann die Koordinaten interpretieren als die Betätigung eines bestimmten Bedienelements auf der betreffenden Bildschirmanordnung.

Wenn dem Anzeigegerät die Position einer Taste bekannt ist, so kann das Anzeigegerät in einigen Ausführungsformen das Betätigen des Touchscreens interpretieren als das Betätigen des bestimmten Bedienfelds und überträgt nicht (oder nicht allein) die Koordinaten der Berührung sondern (auch) das bereits interpretierte Betätigen des entsprechenden Bedienelements. In einigen Ausführungsformen können auch mehrere gleichzeitige Berührungen und/oder Gesten (z.B. Wischen) übertragen werden, beispielsweise bei einer graphischen Benutzerschnittstelle, die das Vergrößern und Verkleinern von Bildschirminhalten, beispielsweise einer Gebrauchsanweisung, mit zwei Fingern ermöglicht (z. B. pinch to zoom). Die derartige Übertragung von mehreren gleichzeitigen Berührungen und/oder Gesten kann somit vorteilhaft insbesondere auch auf Blutbehandlungseinrichtungen möglich sein, die selber über keine Eingabemöglichkeiten verfügen, die mehrere gleichzeitige Berührungen und/oder Gesten erkennen können, wie z. B. Multi-Touchscreen-Displays.

In einigen Ausführungsformen muss das Anzeigegerät zur Überwachung und/oder Fernsteuerung explizit auf der Blutbehandlungseinrichtung für die Fernsteuerung autorisiert werden, alternativ kann eine Autorisierung auch ausschließlich über das Anzeigegerät erfolgen.

In einigen Ausführungsformen sind sowohl auf der Blutbehandlungseinrichtung als auch auf dem Anzeigegerät Autorisierungen erforderlich, um eine Überwachung und/oder eine Fernbedienung zu ermöglichen.

Die Autorisierung kann in einigen Ausführungsformen durch den Bediener erfolgen oder ausschließlich durch den Administrator möglich sein. In einigen Ausführungsformen kann eine Autorisierung für alle verbundenen und/oder verbindbaren Anzeigegeräte erfolgen.

In einigen Ausführungsformen kann eine Autorisierung auf unbestimmte Zeit erfolgen, in anderen sind nur Autorisierungen für begrenzte Zeiträume oder eine begrenzte Zahl von Sitzungen möglich, insbesondere nur für eine Sitzung. Um Fehlbedienungen zu vermeiden kann in einigen Fällen zur Autorisierung auf dem Anzeigegerät der Patientenname auf das Anzeigegerät übertragen werden und/oder dort angezeigt werden.

In einigen Ausführungsformen können bei Fernbedienung die Tonausgaben am Gerät teilweise oder komplett unterbunden werden, so dass ein Patient weiterschlafen kann, wenn eine sonst mit Ton unterlegte Meldung ausgegeben wird. Die Tonausgabe kann stattdessen in einigen Ausführungsformen am Anzeigegerät ausgegeben werden. Beispielsweise könnte auf diese Weise ein Patient schlafen, ohne dass er durch eine Meldung geweckt würde. Störungen könnten dann von dem Überwacher beseitigt werden, in einigen Fällen aus der Ferne.

In einigen Ausführungsformen ist eine Fernbedienung und/oder Überwachung zu jedem beliebigen Zeitpunkt nur von einem Anzeigegerät aus möglich. In anderen Fällen ist dies gleichzeitig von zwei oder mehr Anzeigegeräten möglich.

In einigen Ausführungsformen können von einem Anzeigegerät mehrere Blutbehandlungseinrichtungen überwacht und/oder fernbedient werden. Damit kann ein Benutzer beispielsweise auf einem Computerbildschirm mehrere Blutbehandlungsgeräte zugleich überwachen. Zudem könnte man in einigen Fällen mehrere Blutbehandlungsgeräte, beispielsweise alle Blutbehandlungsgeräte einer Station, von demselben Computer fernbedienen.

In einigen Ausführungsformen kann von den mehreren Blutbehandlungseinrichtungen, die überwacht werden, zu einem beliebigen Zeitpunkt nur eine Blutbehandlungseinrichtung zur Fernbedienung aktiviert werden. So können Verwechslungen, die bei der gleichzeitigen Fernbedienung von mehreren Blutbehandlungseinrichtungen an einem Anzeigegerät entstehen können, vermieden werden.

In anderen Ausführungsformen können mehrere Blutbehandlungseinrichtungen zugleich ferngesteuert werden. Dabei ist es in einigen Ausführungsformen möglich, durch ein Kommando oder eine Bedienungshandlung am Anzeigegerät Vorgänge in mehreren Blutbehandlungseinrichtungen auszulösen, insbesondere bei Blutbehandlungseinrichtungen, die nicht mit Patienten verbunden sind. Eine derartige Fernbedienung, mit der mehrere Blutbehandlungsgeräte zugleich bedient werden, kann vorzugsweise bei Initialisierungen, Selbsttests, Priming, Reinigungen und/oder Abschalten verwendet werden. Auch bei Herstellung oder Wartung könnte die Fernbedienung in einigen Ausführungsformen zum Einsatz kommen. Beispielsweise könnten so mehrere Geräte zugleich getestet werden, was den Personalaufwand in einigen Fällen reduzieren würde. Auch eine Aufzeichnung der Fernbedienung, insbesondere der Tests von einem oder mehreren Blutbehandlungsgeräten oder von einer oder mehreren Blutbehandlungen durch Blutbehandlungsgeräte könnten zentral auf dem Anzeigegerät stattfinden.

In einigen Ausführungsformen können zurückliegende Bildschirmanordnungen erneut angezeigt werden. Die Bildschirmanordnungen können gleichsam wie bei einer Videoaufzeichnung vorzugsweise angehalten, schneller abgespielt und vor- und/oder zurückgespult werden. Dies kann in einigen Ausführungsformen auf dem Bildschirm der Blutbehandlungseinrichtung und/oder auf dem Anzeigegerät geschehen. Dazu können die Bildschirmanordnungsdaten in einigen Ausführungsformen auf dem Blutbehandlungsgerät und/oder auf dem Anzeigegerät gespeichert werden.

In einigen Ausführungsformen, in denen die Bildschirmanordnungsdaten nur auf der Blutbehandlungseinrichtung gespeichert sind, kann dennoch auf vergangene Bildschirmanordnungsdaten zurückgegriffen werden, indem die jeweiligen Daten durch das Anzeigegerät von der Blutbehandlungseinrichtung abgerufen werden.

Das wiederholte Abspielen, schnellere Abspielen, Vor- und/oder Zurückspulen der Bildschirmanordnungen wie bei einem Video kann in einigen Ausführungsformen für eine Usability-Analyse und/oder eine Analyse bei vermuteter Fehlbedienung der Blutbehandlungseinrichtung nützlich sein. Insbesondere, wenn Patienten sich nicht mehr daran erinnern, wie sie das Blutbehandlungsgerät bedient haben, kann ein derartiges erneutes Abspielen der Bildschirmanordnungen bei einer vermuteten oder tatsächlichen Fehlbedienung nützlich sein. Unter anderem für Schulungszwecke oder als Marketing können aufgezeichnete Abfolgen von Bildschirmanordnungen verwendet werden, die sich wie ein Video abspielen lassen.

In einigen Ausführungsformen benötigen Abfolgen von Bildschirmanordnungen nur einen sehr geringen Speicherplatz. Das heißt beispielsweise, dass die kompletten Bildschirmanordnungsdaten einer Blutbehandlung einen Speicherplatzbedarf von nur wenigen Kilobytes besitzen. Ein herkömmliches digitales Video der Bildschirmanordnungen einer Behandlung hat typischerweise einen um mehrere Größenordnungen größeren Speicherbedarf. Auch das Abspeichern von jeder Bildschirmdarstellung als Bilddatei (z.B. PNG, JPG, GIF, TIFF oder BMP) hätte einen erheblich höheren Speicherplatzbedarf.

In einigen Ausführungsformen werden die Bildschirmanordnungsdaten als Textdatei abgespeichert und/oder als Text übertragen. Die Textdatei bzw. der Text ist vorzugsweise als XML formatiert. Vorzugsweise werden die Bildschirmanordnungsdaten in dem zu speichernden Format auf der Blutbehandlungseinrichtung erzeugt, in anderen Ausführungsformen werden sie auf dem Anzeigegerät erzeugt.

In einigen Ausführungsformen werden die Bildschirmanordnungsdaten in einem fest verbauten Speicher auf der Blutbehandlungseinrichtung und/oder dem Anzeigegerät gespeichert. Alternativ oder zusätzlich können die Bildschirmanordnungsdaten auf einem maschinenlesbaren Träger gespeichert werden, wenn dieser mit der Blutbehandlungseinrichtung und/oder dem Anzeigegerät verbunden ist. Die maschinenlesbaren Träger können für Backups verwendet werden oder zur Beweissicherung. Alternativ oder zusätzlich können in einigen Ausführungsformen die Bildschirmanordnungsdaten in ihrer Rohfassung ausgedruckt werden, beispielsweise als Text, insbesondere XML.

Backups der Bildschirmanordnungsdaten können in einigen Ausführungsformen auch über ein mit der Blutbehandlungseinrichtung und/oder dem Anzeigegerät verbundenes Netzwerk durchgeführt werden. Die Bildschirmanordnungsdaten können in einigen Ausführungsformen ein Eventprotokoll darstellen.

Ein Anzeigegerät zur Fernbedienung kann beispielsweise ortsfest angebracht sein, wie z.B. ein speziell nur zur Fernbedienung von einer oder mehreren Blutbehandlungseinrichtungen ausgeführtes Anzeigegerät, beispielsweise mit Touchscreen. In einigen Fällen kann das Anzeigegerät zur Fernbedienung ein ortsfest installierter oder tragbarer Computer sein (insbesondere ein Laptop, Tablet-Computer oder Smartphone), auf dem eine spezielle Anwendung zur Fernbedienung ausgeführt wird oder auf dem ein Browser ausgeführt wird, der in Zusammenwirken mit einem Webserver als Anzeigegerät funktioniert. Ein Webserver kann dabei auf dem Computer, auf dem der Browser ausgeführt wird, ausgeführt werden oder auf der Blutbehandlungseinrichtung. In anderen Ausführungsformen kann der Webserver auch auf einem dritten Gerät ausgeführt werden, beispielsweise auf einem dedizierten Server.

In einigen Ausführungsformen kann ein Patient die Blutbehandlungseinrichtung, an die er z.B. gerade angeschlossen ist, über ein Anzeigegerät zur Fernbedienung bedienen, beispielsweise über einen Tablet-Computer oder ein Smartphone. Wenn beispielsweise ein Patient bei einer Behandlung mit einem Blutbehandlungsgerät von diesem durch einen Meldungstext (z.B. einen Alarm) geweckt wird, so kann er die Meldung mit dem Tablet-Computer oder Smartphone quittieren ohne aufstehen zu müssen. Auch könnte in einigen Fällen die Meldung für den Patienten einfacher zu lesen sein, wenn sein Anzeigegerät einen größeren Bildschirm als die Blutbehandlungseinrichtung aufweist oder es erlaubt, den Meldungstext zu vergrößern. In diesen und anderen Fällen könnte das Anzeigegerät mit der Blutbehandlungseinrichtung über Bluetooth oder Wifi verbunden sein.

In einigen Ausführungsformen könnte die Blutbehandlungseinrichtung eine Dockingstation für einen tragbaren Computer, wie z.B. einen Tablet-Computer oder ein Smartphone, besitzen. Der Benutzer könnte seinen tragbaren Computer dort verwahren und in einigen Ausführungsformen dort aufladen. Zudem ist es so in einigen Ausführungsformen möglich, auf einen Bildschirm an der Blutbehandlungseinrichtung zu verzichten und den tragbaren Computer als das einzige Anzeigegerät zu verwenden.

In einigen Ausführungsformen werden die Bildschirmanordnungen und/oder Abfolgen von Bildschirmanordnungen, die auf der Blutbehandlungseinrichtung angezeigt werden, exakt auf dem Anzeigegerät reproduziert. Dazu kann z.B. das Anzeigegerät Prüfdaten an die Blutbehandlungseinrichtung übertragen, aus denen ermittelt werden kann, ob die Bildschirmanordnungen identisch sind. In einigen Ausführungsformen kann dabei ein Hashwert oder eine Prüfsumme der Bildschirmanordnung berechnet werden. Der Hashwert bzw. Prüfsumme kann dann von dem Anzeigegerät auf die Blutbehandlungseinrichtung (oder umgekehrt) übertragen werden und auf der Blutbehandlungseinrichtung und/oder dem Anzeigegerät überprüft werden. So kann überprüft werden, ob die Bildschirmanordnungen auf den unterschiedlichen Geräten identisch sind.

In einigen Ausführungsformen kann durch die Überprüfung des Hashwerts bzw. der Prüfsumme ein Nachweis erbracht werden, ob zwei Bildschirmanordnungen, die auf unterschiedlichen Geräten angezeigt werden, identisch sind, insbesondere in jedem Pixel identisch sind. Dies könnte beispielsweise bei sicherheitskritische Applikationen relevant sein. Der Hashwert bzw. die Prüfsumme könnten auch beispielsweise verwendet werden, um die Identität der Bildschirmanordnungen im Rahmen der geltenden Gesetze zu dokumentieren und für einen rechtlichen Nachweis aufzuzeichnen.

In einigen Ausführungsformen kann die Erfindung u. a. die folgenden Vorteile aufweisen:
Eine exakte Rekonstruktion, und keine ähnliche Nachbildung, kann wichtig für die Sicherheit und Zuverlässigkeit einiger Ausführungsformen sein und eine Verifikation in besonderem Maße erleichtern.

Im Folgenden wird die vorliegende Erfindung unter Bezugnahme auf die beigefügte, stark vereinfachte Zeichnung beschrieben. Es zeigen:
Fig. 1 eine Blutbehandlungseinrichtung und ein Anzeigegerät;
Fig. 2 ein Menü mit zwei Einträgen;
Fig. 3 eine Blutbehandlungseinrichtung, die von einer Anzeigeeinrichtung fernbedient wird;
Fig. 4 eine Rekonstruktion einer Bildschirmanordnung
   und
Fig. 5 schematisch vereinfacht eine beispielhafte erfindungsgemäße Blutbehandlungseinrichtung mit einer Steuervorrichtung in einer exemplarischen Ausführungsform.

Die vorliegende Erfindung ist nicht auf die vorstehend beschriebenen Ausführungsformen beschränkt, diese dienen lediglich der Veranschaulichung.

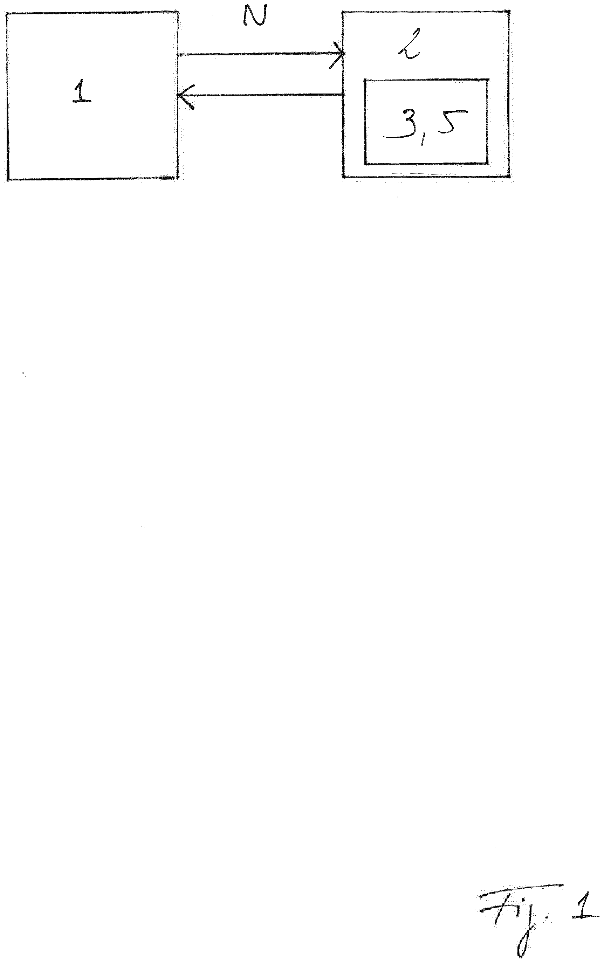

Fig. 1 zeigt eine Blutbehandlungseinrichtung 1, die über ein drahtloses Netzwerk N an ein Anzeigegerät 2 mit einem Bildschirm 3, der als Touchscreen 5 ausgeführt ist, angebunden ist. Ein Patient ist zur Blutbehandlung an der Blutbehandlungseinrichtung 1 angeschlossen. Eine Krankenschwester kann über das Anzeigegerät 2 die Blutbehandlung überwachen und die Blutbehandlungseinrichtung 1 aus der Ferne über das Anzeigegerät 2 bedienen.

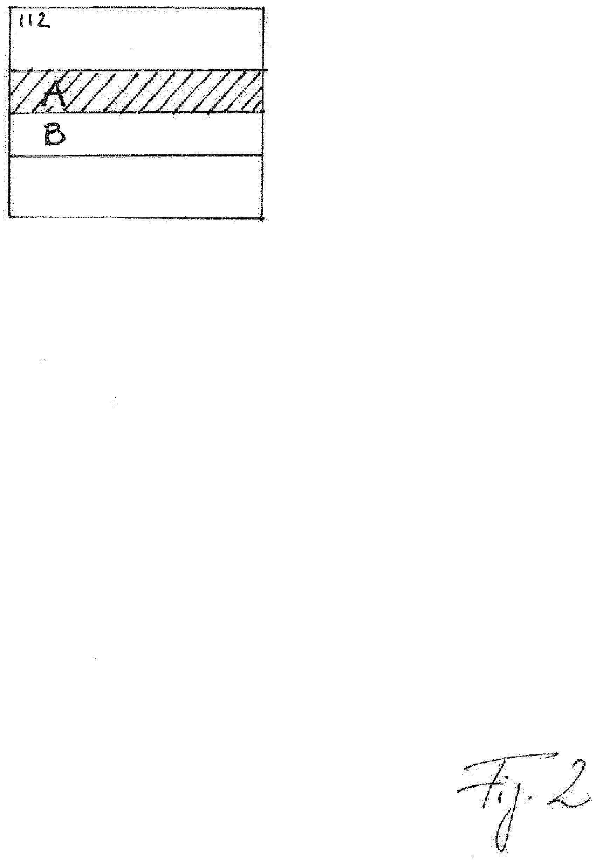

Fig. 2 zeigt in ein Menü mit zwei Einträgen (Einträge A und B). In jedem Menüeintrag existiert ein Button, der den Zustand gedrückt (pressed) und "nicht gedrückt" (not pressed) besitzen kann. Um einen Zustand des Menüs beispielsweise aus gespeicherten Daten rekonstruieren zu können, benötigt man die Bildschirmnummer, die momentane Menüposition und den Buttonstatus. Im hier gezeigten Beispiel wurde der Bildschirm (mit einer definierten Bildschirmnummer, hier 112) derart bedient, dass er sich in der Menüposition 0 befindet (die der Zeile entspricht, in der Eintrag A dargestellt ist) und der entsprechende Button für Eintrag A gedrückt ist (der Status des Buttons ist dann Buttonstatus=pressed).

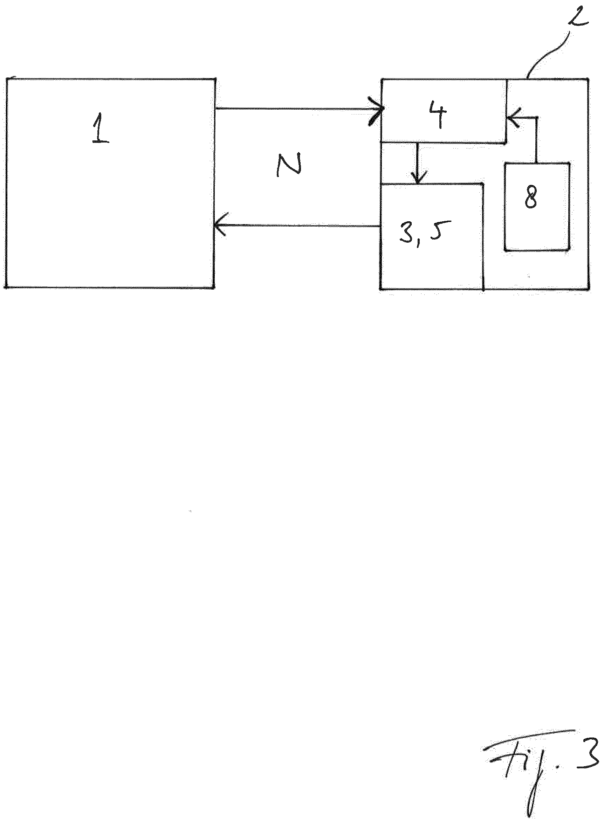

Fig. 3 zeigt eine Blutbehandlungseinrichtung 1 und ein Anzeigegerät 2 zur Verwendung in dem erfindungsgemäßen Verfahren zur Überwachung und Fernbedienung. In Fig. 3 stellt die Blutbehandlungseinrichtung 1 über ein Netzwerk N dynamische Daten an das Anzeigegerät 2 bereit. Die dynamischen Daten werden zusammen mit GUI-Layout-Ressourcen 8 von einem Interpreter 4 in dem Anzeigegerät 2 interpretiert und als Bildschirmanordnung auf einem Bildschirm 3 (hier ausgeführt als Touchscreen 5) des Anzeigegeräts 2 ausgegeben. Wenn ein Benutzer zur Fernbedienung eine Eingabe am Touchscreen 5 des Anzeigegeräts macht, dann wird die entsprechende Information, beispielsweise die Koordinaten der Berührung auf dem Touchscreen 5 und/oder eine Identifikation eines auf dem Touchscreen 5 dargestellten virtuellen Buttons an die Blutbehandlungseinrichtung 1 übermittelt. Die übermittelten Informationen veranlassen in einigen Fällen die Blutbehandlungseinrichtung 1 zu bestimmten programmierten Aktionen.

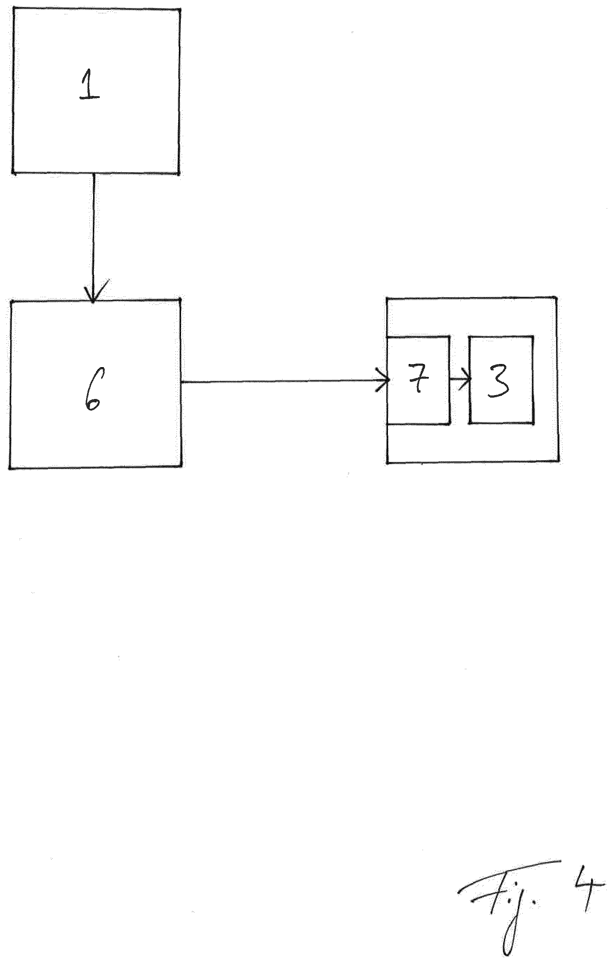

In Fig. 4 werden die Bildschirmanordnungen, die während einer Blutbehandlung von einer Blutbehandlungseinrichtung 1 angezeigt werden, als Bildschirmanordnungsdaten auf einem maschinenlesbaren Träger 6, beispielsweise einer SD-Karte oder einer CD, gespeichert. Zu einem späteren Zeitpunkt können die Bildschirmanordnungsdaten von einem Anzeigegerät 2 aus dem maschinenlesbaren Träger 6 abgerufen werden. Die Bildschirmanordnungen können dann aus den Bildschirmanordnungsdaten mit Hilfe des Visualisierers 7 (der hier in Software ausgeführt ist) rekonstruiert und die Bildschirmanordnungen auf dem Bildschirm 3 des Anzeigegeräts 2 angezeigt werden. Die Bildschirmanordnungen können in der tatsächlichen Geschwindigkeit der stattgefundenen Blutbehandlung angezeigt werden, schneller abgespielt, angehalten, vor- und zurückgespult werden. Alternativ können die Bildschirmanordnungsdaten auch von der Blutbehandlungseinrichtung 1 ausgelesen werden und rekonstruiert werden. Dazu weist die Blutbehandlungseinrichtung 1 vorzugsweise ebenfalls einen Visualisierer 7 auf.

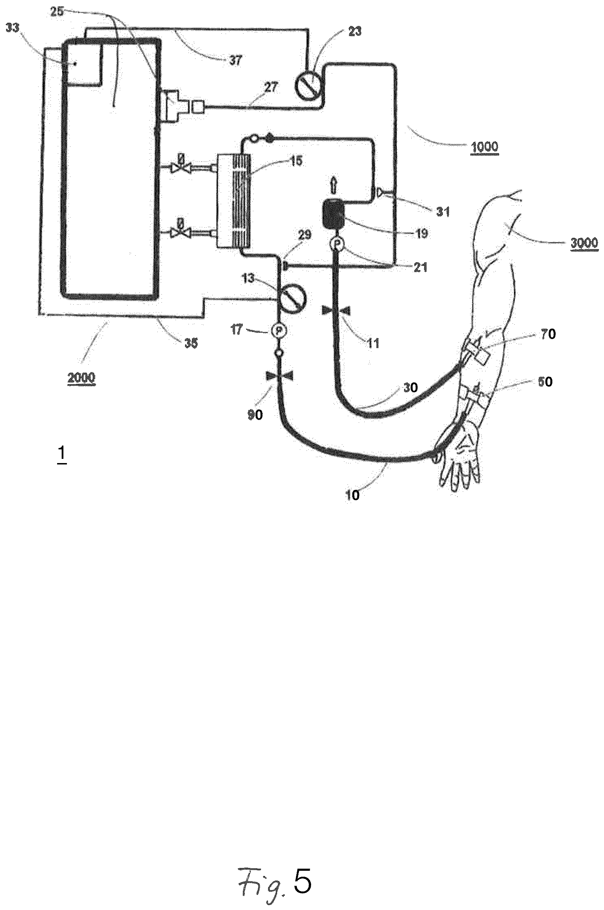

Fig. 5 zeigt eine erfindungsgemäße Blutbehandlungseinrichtung 1, die rein exemplarisch eine Dialyseeinrichtung 2000 ist. Auch aus diesem Grund sind die im Folgenden genannten Komponenten der Blutbehandlungseinrichtung 1 daher jeweils als optional zu verstehen.

In Fig. 5 ist ein extrakorporaler Blutkreislauf 1000, verbunden mit einer erfindungsgemäßen Blutbehandlungseinrichtung 1, mit einem Double-Needle-Zugang zum Gefäßsystem eines Patienten 3000 dargestellt. Der extrakorporale Blutkreislauf 1000 weist einen arteriellen Leitungsabschnitt 10 sowie einen venösen Leitungsabschnitt 30 auf.

Der arterielle Leitungsabschnitt 10 ist über einen arteriellen Patientenzugang, beispielsweise, wie in Fig. 5 gezeigt, in Form einer arteriellen Konnektionsnadel 50, mit dem Gefäßsystem des Patienten 3000 verbunden. Der venöse Leitungsabschnitt 30 ist über einen venösen Patientenzugang, beispielsweise, wie in Fig. 5 gezeigt, in Form einer venösen Konnektionsnadel 70, mit dem Gefäßsystem des Patienten 3000 verbunden.

Der arterielle Leitungsabschnitt 10 weist eine arterielle Patientenschlauchklemme 90 auf, der venöse Leitungsabschnitt 30 weist eine venöse Patientenschlauchklemme 11 auf, oder ist hiermit jeweils verbunden.

Der extrakorporale Blutkreislauf 1000 ist in eine Blutpumpe 13 der Blutbehandlungseinrichtung 1 eingelegt. Die Blutpumpe 13 ist exemplarisch als Rollenpumpe ausgestaltet. Die Blutpumpe 13 fördert Blut des Patienten 3000 durch den extrakorporalen Blutkreislauf 1000.

Der extrakorporale Blutkreislauf 1000 weist eine Blutbehandlungseinrichtung 15, beispielsweise einen Hämodialysator, Blutfilter oder dergleichen, auf.

Wie in Fig. 5 gezeigt, ist am arteriellen Leitungsabschnitt 10 ein arterieller Drucksensor 17 stromabwärts (bezogen auf die während einer Blutbehandlung übliche Förderrichtung) von der Blutpumpe 13 angeordnet.

Im venösen Leitungsabschnitt 30 sind eine venöse Tropfkammer 19 und ein venöser Drucksensor 21 stromaufwärts der venösen Tropfkammer 19 enthalten oder angeordnet.

Mittels einer zweiten optionalen Fördereinrichtung, beispielsweise einer Substituatpumpe 23, kann Substituatflüssigkeit aus einer Substituatflüssigkeitsvorratseinrichtung 25 über eine Substituatflüssigkeitszuleitung 27 an einer Prädilutions-Zugabestelle 29 für Substituatflüssigkeit und/oder einer Postdilutions-Zugabestelle 31 für Substituatflüssigkeit in den extrakorporalen Blutkreislauf 1000 eingebracht werden. Die Substituatpumpe 23 ist hier exemplarisch als Rollenpumpe ausgestaltet.

Die Blutbehandlungseinrichtung 1 weist eine Signalverarbeitungseinheit 33 auf. Die Signalverarbeitungseinheit 33 ist über eine Steuerleitung 35 mit der Blutpumpe 13 und über eine Steuerleitung 37 mit der Substituatpumpe 23 verbunden.

Die Signalverarbeitungseinheit 33 kann beispielsweise eine Förderrate oder eine Förderleistung der Blutpumpe 13 in Abhängigkeit von Betriebsparametern der Substituatpumpe 23, z. B. deren Förderrate oder Förderleistung, steuern.

Die Steuerleitungen 35 und 37 können eine bidirektionale Kommunikation zwischen der Signalverarbeitungseinheit 33 und der Blutpumpe 13 bzw. der Substituatpumpe 23 zulassen.

Fig. 5 zeigt eine Verbindung der erfindungsgemäßen Blutbehandlungseinrichtung 1 mit dem Gefäßsystem des Patienten 3000. Eine solche Verbindung ist nicht Teil der erfindungsgemäßen Blutbehandlungseinrichtung 1, sondern stellt nur deren besondere Ausgestaltung und bevorzugte Nutzung dar.

### Bezugszeichenliste

- 1: Blutbehandlungseinrichtung
- 2: Anzeigegerät
- 3: Bildschirm
- 4: Interpreter
- 5: Touchscreen
- 6: maschinenlesbarer Träger
- 7: Visualisierer
- 8: GUI-Layout-Ressourcen
- 10: arterieller Leitungsabschnitt
- 30: venöser Leitungsabschnitt
- 50: arterielle Konnektionsnadel
- 70: venöse Konnektionsnadel
- 90: arterielle Patientenschlauchklemme
- 11: venöse Patientenschlauchklemme
- 13: Blutpumpe
- 15: Blutbehandlungseinrichtung
- 17: arterieller Drucksensor
- 19: venöse Tropfkammer
- 21: venöser Drucksensor
- 23: Substituatpumpe
- 25: Substituatflüssigkeitsvorratseinrichtung
- 27: Substituatflüssigkeitszuleitung
- 29: Prädilutions-Zugabestelle

- 31: Postdilutions-Zugabestelle

- 33: Signalverarbeitungseinheit
- 35: Steuerleitung zur Blutpumpe
- 37: Steuerleitung zur Substituatpumpe
- 1000: extrakorporaler Blutkreislauf
- 2000: Dialyseeinrichtung
- 3000: Patient

## Patentansprüche

1. Verfahren zur Überwachung und/oder Fernbedienung einer Blutbehandlungseinrichtung (1), die folgenden Schritte umfassend:
- Bereitstellen eines Anzeigegeräts (2), wobei das Anzeigegerät (2) einen Bildschirm (3) und einen Interpreter (4) aufweist;
- Bereitstellen von GUI-Layout-Ressourcen (8) und von dynamischen Daten;
- Interpretation der GUI-Layout-Ressourcen (8) und der dynamischen Daten durch den Interpreter (4);
- Ausgabe einer Bildschirmanordnung durch den Interpreter (4);
- Anzeigen der Bildschirmanordnung auf dem Bildschirm (3).

2. Verfahren nach Anspruch 1, wobei die GUI-Layout-Ressourcen (8) auf dem Anzeigegerät (2) vorgehalten und/oder über ein mit dem Anzeigegerät (2) verbundenes Netzwerk (N) bereitgestellt werden.

3. Verfahren nach einem der vorhergehenden Ansprüche, wobei die dynamischen Daten von der Blutbehandlungseinrichtung (1) über eine kabelgebundene Verbindung oder eine kabellose Verbindung, insbesondere ein kabelgebundenes oder ein kabelloses Netzwerk (N), bereitgestellt werden.

4. Verfahren nach einem der vorhergehenden Ansprüche, wobei das Anzeigegerät ein oder mehrere Eingabeelemente umfasst, insbesondere Tasten, Schalter und/oder einen Touchscreen (5), wobei das Verfahren umfasst: Übertragen einer von einem Bediener über das Eingabeelement des Anzeigegeräts (2) getätigten Eingabe an die Blutbehandlungseinrichtung (1), insbesondere über das Netzwerk (N).

5. Verfahren nach einem der vorhergehenden Ansprüche, wobei das Anzeigegerät (2) zur Überwachung und/oder Fernbedienung auf der Blutbehandlungseinrichtung (1) autorisiert werden muss.

6. Verfahren, die folgenden Schritte umfassend:
- Bereitstellen einer Blutbehandlungseinrichtung (1) mit einem Bildschirm (3);
- Bereitstellen eines maschinenlesbaren Trägers (6) und/oder eines Netzwerkes (N);
- Speichern von Informationen über den Zustand der Blutbehandlungseinrichtung (1) auf dem maschinenlesbaren Träger (6) und/oder Übertragen der Daten in das Netzwerk (N), wobei die Informationen Bildschirmanordnungsdaten umfassen, aus denen eine oder mehrere Bildschirmanordnungen der Blutbehandlungseinrichtung (1) rekonstruiert werden können.

7. Verfahren nach Anspruch 6, wobei die Informationen als eine Textdatei gespeichert und/oder als Text übertragen werden, insbesondere im XML-Format.

8. Verfahren zum Rekonstruieren, insbesondere zum exakten Rekonstruieren, einer oder einer Abfolge mehrerer Bildschirmanordnungen aus einer Blutbehandlungseinrichtung (1), die folgenden Schritte umfassend:
- Bereitstellen eines Visualisierers (7);
- Bereitstellen von Bildschirmanordnungsdaten von einem maschinenlesbaren Träger (6) und/oder aus einem Netzwerk (N);
- Verarbeiten der Bildschirmanordnungsdaten durch den Visualisierer (7); und
- Ausgabe einer Bildschirmanordnung;
- Anzeigen der Bildschirmanordnung oder der Abfolge von Bildschirmanordnungen auf einem Bildschirm (3) des Anzeigegeräts (2) oder der Blutbehandlungseinrichtung (1).

9. Verfahren nach Anspruch 8, wobei das Anzeigen der Abfolge von Bildschirmanordnungen ein schnelleres Abspielen der Bildschirmanordnungen und/oder ein Zurück- und/oder Vorspulen erlaubt.

10. Verfahren nach einem der Ansprüche 8 oder 9, wobei die Bildschirmanordnungsdaten durch das Verfahren nach Anspruch 6 und 7 erzeugt wurden.

11. Anzeigegerät (2) zur Überwachung und/oder Fernbedienung einer Blutbehandlungseinrichtung (1), geeignet und/oder konfiguriert zum Ausführen des Verfahrens nach Anspruch 1 bis 10.

12. Blutbehandlungseinrichtung (1), geeignet und/oder konfiguriert zum Ausführen des Verfahrens nach Anspruch 1 bis 10.

13. Digitales Speichermedium, insbesondere in Form einer Diskette, CD oder DVD oder EPROM, mit elektronisch auslesbaren Steuersignalen, konfiguriert, um derart mit einem programmierbaren Computersystem zusammenzuwirken, dass die maschinellen Schritte eines erfindungsgemäßen Verfahrens nach einem der Ansprüche 1 bis 10 veranlasst werden.

14. Computerprogramm-Produkt mit einem auf einem maschinenlesbaren Träger gespeicherten Programmcode zur Veranlassung der maschinellen Schritte des erfindungsgemäßen Verfahrens gemäß einem der Ansprüche 1 bis 10, wenn das Computerprogramm-Produkt auf einem Computer abläuft.

15. Computerprogramm mit einem Programmcode zur Veranlassung der maschinellen Schritte eines erfindungsgemäßen Verfahrens gemäß einem der Ansprüche 1 bis 10, wenn das Computerprogramm auf einem Computer abläuft.
